# EUROPEAN PATENT APPLICATION

(11) **EP 4 169 574 A1**
(43) Date of publication of application: **26.04.2023**
(21) Application number: 21382944.3
(22) Date of filing: 20.10.2021
(51) Int. Cl.: A61N 5/10, A61B 90/00, A61B 5/113, A61B 5/00

(54) **PATIENT POSITIONING SYSTEM FOR RADIATION EMISSION TREATMENT AND/OR DIAGNOSIS EQUIPMENT**

(71) Applicant: Fundació Institut d'Investigació Biomèdica de Girona Dr. Josep Trueta (IDIBGI), 17190 Salt (Girona) (ES); Institut Català d'Oncologia (ICO), 08908 L'Hospitalet de Llobregat (ES); Compoxi SL, 17003 Girona (ES)
(72) Inventor: ROMERA MARTÍNEZ, Ingrid, 17007 Girona (ES); MUÑOZ MONTPLET, Carles, 17007 Girona (ES); JURADO BRUGGEMAN, Diego, 17007 Girona (ES); MARRUECOS QUEROL, Jordi, 17007 Girona (ES); BOU SÁNCHEZ, Salvador, 17007 Girona (ES); GRÈBOL CANAL, Roger, 17007 Girona (ES); GASCONS TARRÉS, Marc, 17003 Girona (ES); VICENS CUYÀS, Pep, 17003 Girona (ES)
(74) Representative: Hoffmann Eitle

(57) **Abstract**

The invention refers to a system for positioning patients during treatment and/ or diagnosis processes, preferably for radiotherapy treatments, in which patients must be irradiated in a deep inspiration breath hold position. The system is synchronized with an electron accelerator, in a way that radiation is applied on a patient only if the patient is in a proper deep inspiration hold position. The electron accelerator is controlled by the contact sensor (12), preferably a pressure sensor, so that radiation can be applied on a patient only when a correct pressure is exerted on the contact sensor (12). In addition, while the contact sensor is pressed correctly, signalling means are activated, to inform a patient and an accelerator operator, that the patient is in a proper deep inspiration hold position. The invention achieves high reproducibility in patient positioning while irradiating patients in deep inspiration breath hold position, and avoiding undesired radiation to other vital organs such as the heart.

## Description

### Field and object of the invention

The invention refers in general to apparatus for correctly positioning patients during treatment and / or diagnosis processes, preferably for medical processes wherein patients must be irradiated in a deep inspiration breath hold position, maintaining said position during the irradiation process, as for example: treatments for left breast cancer, supradiaphragmatic lymphoma, or extracranial stereotactic techniques in the lung, liver and pancreas.

An object of the invention is to provide a simple to use positioning system of the above-mentioned type, that provides a direct physical reference of the thoracic amplitude of a patient with no need of expensive equipment that requires complex interpretation.

The medical device of the invention provides a physical reference of the movement of a patient in deep inspiration breath hold, such that the device is synchronized with an electron linear accelerator, in a way that the electron accelerator is directly controlled by that physical reference, thereby enhancing precision of the radiotherapy treatment and reliability.

The medical device includes a control module that is portable and versatile in the sense that it can be used with any CT and any accelerator, assuring interchangeability in any CT or any accelerator if necessary.

The system is preferably adapted for its use in radiotherapy treatments.

### Background of the invention

The cardiotoxicity caused by radiotherapy treatments, particularly in treating left breast cancer, is well documented. The risk begins within a few years after radiotherapy treatment and continues for decades. The most recent data [Sara C. Darby et al: Risk of Ischemic Heart Disease in Women after Radiotherapy for Breast Cancer. New England Journal of Medicine; 368:987-98] suggest that the incidence of severe coronary toxicity increases linearly by 7.4% for each gray of mean cardiac dose, with no apparent dose threshold below which no toxicity is detected.

The implementation of radiotherapy techniques that reduce the dose that the heart receives is needed and of outmost importance.

In radiotherapy treatments in which the patient must be irradiated in the Deep Inspiration Breath Hold Irradiation (DIBHI) position, the thoracic cavity expands and the anterior thoracic wall moves in the anterolateral direction. Furthermore, the diaphragm contracts and, as a result, the heart moves backwards and towards the diaphragm. These modifications in the position of the organs increase the distance between the heart and the volume of the irradiation dose, which reduces the dose received by the heart.

All radiotherapy techniques that use breathing control to reduce toxicity to the heart are based on these arguments, since they do not affect the treatment in any way, as long as it may be repeated. In this way they help reduce toxicity to the heart.

To control breathing, in some treatments patients are trained in deep breathing techniques and in holding their breath for up to 30 seconds. This process is usually repeated 3 to 6 times per visit. Currently, in order to ensure that the patient has sufficient deep breathing capacity at the moment of irradiation, there are devices on the market, which emit and detect infrared radiation, among others. The devices based on the emission and detection of infrared radiation are positioned over the patient and synchronized with the electron accelerator to emit radiation only when the patient is in the correct position, previously defined by software provided by the manufacturer of the device. In order for these devices to function properly, a custom adjustment of the position of the infrared emitter is required for each patient, with the aim of correctly synchronizing the same with the electron accelerator.

International PCT publication WO 2018/024630 A1 describes a device for positioning patients in radiotherapy treatments, that comprises a positioning arm capable of varying the distance with respect to a support base configured to receive a patient. During the clinical validation of the device, a redundant infrared reflective device fitted to the positioning arm was used, to synchronize the device with an electron accelerator. However, in this PCT publication no synchronization with an electron accelerator during the normal use of the device in patients, was disclosed or suggested.

### Summary of the invention

The present invention is defined in the attached independent claim, and satisfactorily solves the above-described drawbacks of the prior art, by providing a reference system for positioning of patients in deep inspiration hold in radiotherapy treatments, which provides feedback to both a patient and technical staff, and which is synchronized with an electron accelerator, in a way that radiation is applied on a patient only if the patient is in a proper deep inspiration hold position. The invention achieves high reproducibility in patient positioning, while irradiating a local area of patients in deep inspiration breath hold position and avoiding undesired radiation to other vital organs such as the heart.

The system of the invention generates a direct indication (it does not require interpretation), both for a patient and for an operator of a radiation emission equipment, as to whether the patient is properly holding the breath. In this way, the system does not require expensive ancillary devices for a signal interpretation, thus, the system design and manufacturing cost, are significantly reduced.

Therefore, an aspect of the invention refers to a system for positioning patients in a radiation emission equipment, wherein the system comprises an arm configured to be attached to a medical table of the equipment by means of a support, and a bar mounted with the arm in a displaceable manner so that the position of the bar can be set for each patient.

The bar is generally parallel to the medical table when the arm is coupled to the table, so the height of the bar with respect to the table, is adjustable.

A contact sensor, preferably a pressure sensor, extends longitudinally along the bar, and it is adapted such that when the device is in use, that is, when the arm is attached to the table, the contact sensor is pressed by the thoracic expansion of a patient lying on a table while the patient is in deep inspiration breath hold.

In a preferred embodiment, the bar has a trapezoidal cross-sectional configuration having an upper and lower bases, and the contact sensor is provided on the lower base of the trapezoidal support. The trapezoidal cross-sectional configuration improves contact between the patient and the contact sensor while ensuring appropriate robustness of the bar.

The system further comprises signalling means configured to provide feedback for a patient lying on the table, and a control unit communicated with the contact sensor and with the signalling means. The control unit is adapted to operate the signalling means as to inform a patient lying on the table, whether the contact sensor is properly activated or not.

The system is further adapted to be in communication with a simulation/tumour localisation area or CT zone.

There are two signals to provide feedback, one for the patient and one for the radiotherapy technician or operator at the CT zone. The signal for the patient is acoustic and the signal for the radiotherapy technician and personnel controlling the machine and the treatment from cameras, is luminous. In other embodiments of the invention, there are acoustic and luminous signals for the patient, and also acoustic and luminous signals for the technician.

In this way, only when a patient is in a proper deep inspiration breath hold position and the contact sensor is pressed correctly, the signalling means are activated, so that a patient perceives a sound indicating that situation. As soon as the deep inspiration breath hold position is not correct, the signalling means for the patient and for the radiotherapy operators, indicate that the patient position is not correct, thus, the position can be corrected to a proper deep inspiration breath hold position.

The system is adapted to be communicated with a radiation emission equipment, for example a radiotherapy treatment equipment, in order to control the equipment based on whether the contact sensor is properly pressed or not. Preferably, the contact sensor is a pressure sensor, and a pressure range is predefined for each patient if required, within which it is considered that the patient is in proper deep inspiration breath hold position. The predefined pressure range ensures a preset range of thoracic amplitudes.

In the simulation CT zone, a graphic interface might be provided to define a correct pressure range for each patient.

In this way, the system is synchronized with the radiation emission equipment, such that radiation is applied on a patient only when a patient in deep inspiration breath hold position, is pressuring the pressure sensor within the correct pressure range.In this way, it is assured that radiation is exclusively directed towards the target volume of the patient's body, avoiding damaging other nearby organs, and minimizing cardiotoxicity caused by radiotherapy treatments.

Some advantages of the invention are listed below:
- enhanced radiotherapy treatment with maximum reliability due to the synchronization with the electron accelerator, based on direct transmission of the signal received from the pressure sensor to the accelerator,
- it facilitates work of the nursing and technical staff, because it is easy to place on an equipment table,
- friendly use for patients and technical staff. It provides color-coded feedback with different sounds for easy understanding by patients and technicians. Due to the perceived pressure signal control capability, it provides more precision in patient position,
- versatility of the device and the portable control module that can be used with any CT and any accelerator, assuring interchangeability of devices in any CT or any accelerator if necessary,
- it is comfortable for patients, because it does not require belts or other uncomfortable fixation or invasive devices for patients.

### Brief description of the drawings

Preferred embodiments of the invention are henceforth described with reference to the accompanying drawings, wherein:
Figure 1.- shows a perspective view of the system of the invention.
Figure 2.- shows an elevation view of the system of Figure 1, with the bar represented at an upper position in broken lines.
Figure 3.-shows in Figure 3A a partially sectioned elevational view of the system of Figure 1, including enlarged details. Figure 3B is a top plan view.
Figure 4.- shows a cross-sectional view of the bar.
Figure 5.- shows an electric diagram of the general electronic system.
Figure 6.- shows an electric diagram of the control module.
Figure 7.- shows a general electric diagram of the therapy zone, and simulation zone (CT) controlled by an operator equipment.

### Preferred embodiment of the invention

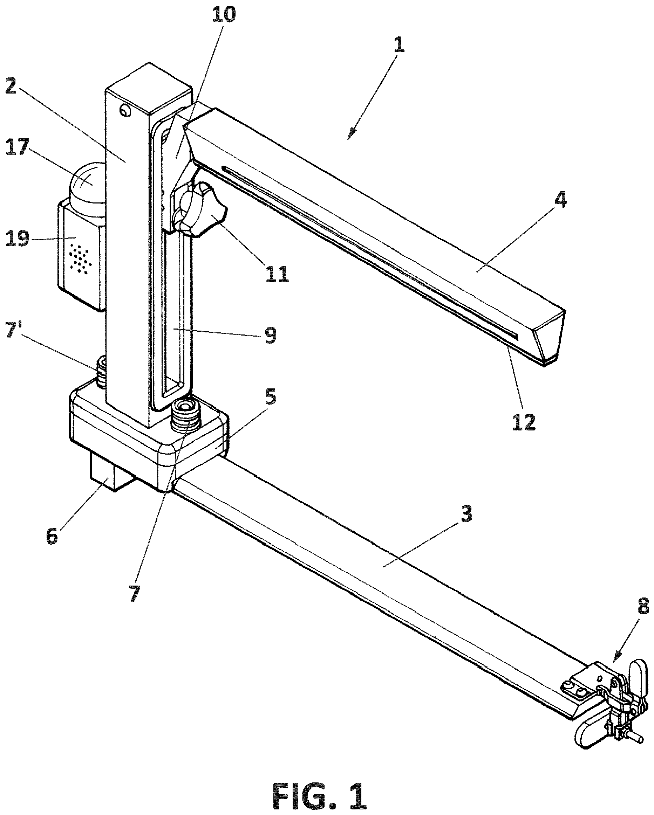

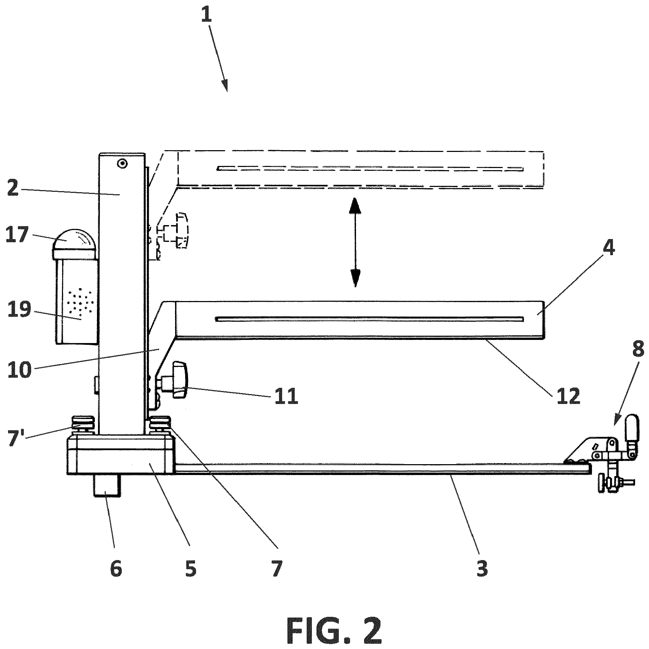

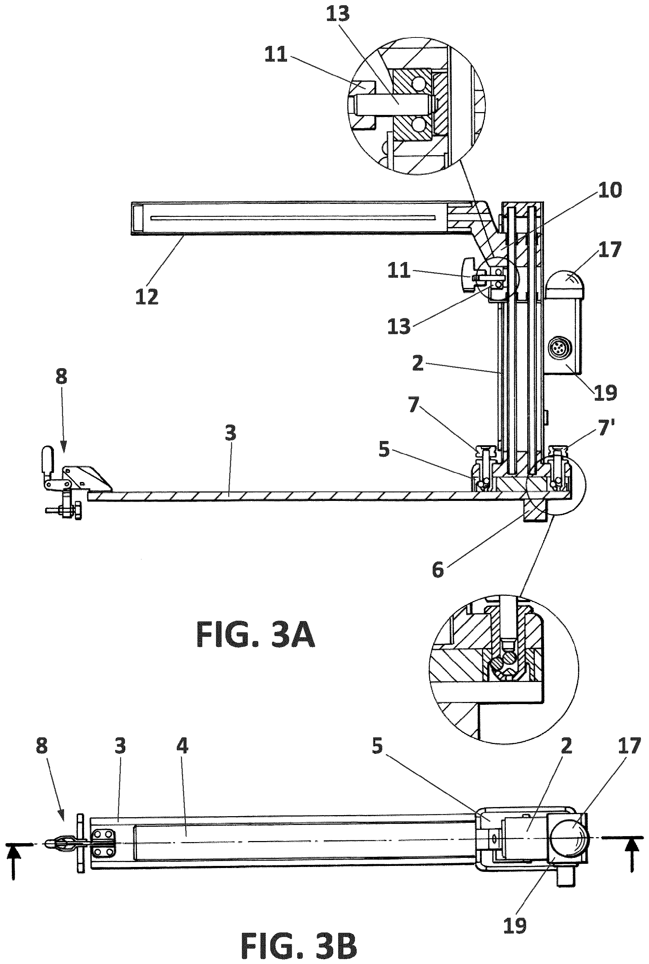

**Figures 1** to 3 show a preferred embodiment of the system (1) of the invention, that comprises an arm (2) configured to be attached to a table of a radiation emission equipment (not shown), by means of a table support (3).

The arm (2) and the table support (3) are straight elongated bodies, orthogonally arranged with respect to each other. In particular, the arm and the table support (2,3) have one end attached to a support base (5), which has a fixation (6) at its lower base, and two clamps (7,7') for fixing the table support (3) with the arm (2). The other end of the table support (3), is fitted with a fixation mechanism (8) for temporarily fixing the table support (3) to a table, that would be received and pressed between the fixation (6) and the fixation mechanism (8).

The system (1) further includes a bar (4) mounted with the arm (2) and displaceable with respect to the arm (2), such that the distance or height of the bar (4) with respect to the table support (3) or a table, can be set for each particular patient.

The arm (2) has an interior channel (9), into which a base (10) of the bar (4) is inserted, such that the channel (9) serves as a guide for the displacement of the bar (4). A knob (11) is coupled to a screw (13) which passes through the base (10) for fixing the bar (4) in a desired position, by pressing the base (10) against the arm (2).

As represented in **Figures 1** to **3B****,** the signalling means comprises an acoustic indicator (19) and/or a luminous indicator (17), both preferably mounted on the arm (2).

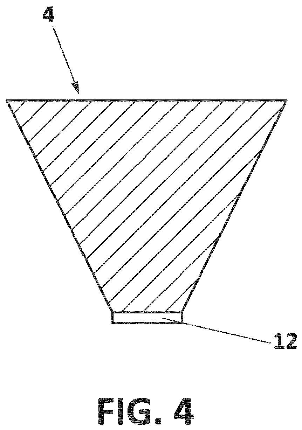

As shown in **Figure 4****,** in this preferred embodiment, the bar (4) has a trapezoidal cross-sectional configuration having an upper and lower bases, and the contact sensor (12) is provided on the lower base of the trapezoidal bar (4). The lower base provides a flat supporting surface for mechanically fixing the contact sensor.

Preferably, most of the components of the system are manufactured with Carbon Fiber Reinforced Plastic (CFRP), or other suitable material that does not interfere with radiation.

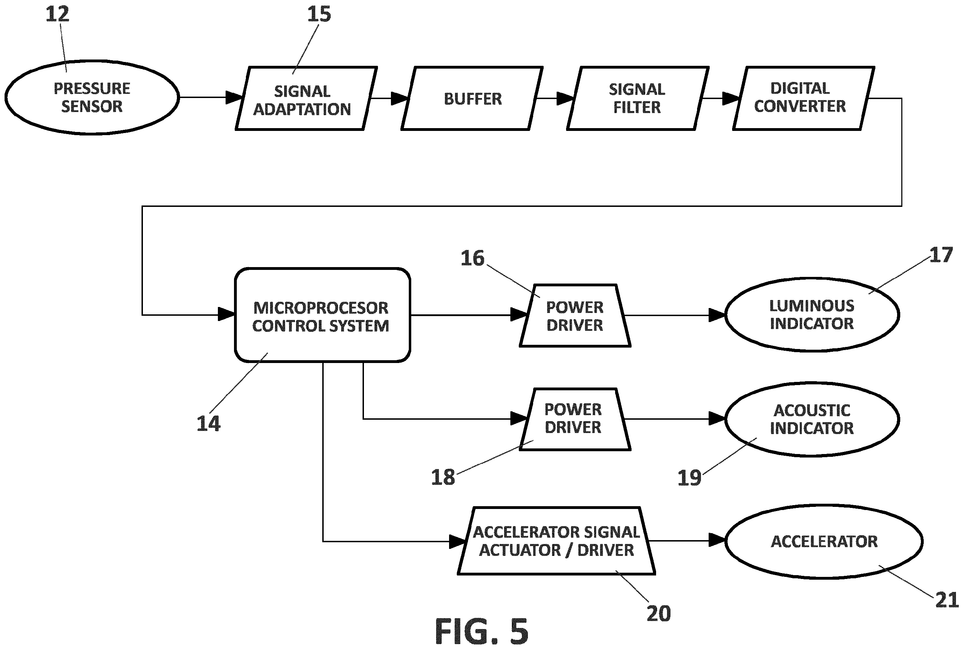

**Figure 5** shows an electric diagram of the general electronic system for processing the analogue signal generated by the contact sensor (12), preferably implemented as a pressure sensor, upon activation by a patient. The analogue signal generated by the pressure sensor is adapted at a signal adaptation device (15), which it is adapted to the desired needs and behavior. The system includes a variable resistance to be able to fine-tune the linearity and accuracy of the resulting signal, which passes through a buffer to maintain the signal level and isolate it from the rest of the circuit, in order to prevent the signal from being affected by the electronics of the system.

The signal then passes through a low-pass filter for its stabilization in the event of sudden variations, in order to be able to have a better reading. At this stage, the filter output is already suitable for analogue/digital conversion at an analog/digital converter, for example a 10-bit resolution converter.

A control unit includes a microprocessor control system (14) communicated with the analog/digital converter, and reads the digital signal. After the reading, the microprocessor control system (14) performs a classification, depending on whether it is a very low, low, adequate or high value, and acts accordingly. If it is a very low value, the system does not activate any indicator, neither luminous nor acoustic. If it is a low value, the system activates a blue light indicator and the acoustic indicator generates a low frequency sound, indicating that more pressure is needed.

If the measured pressure is within a predefined range of adequate pressures, the system activates a green indicator light and the acoustic indicator generates a medium frequency sound, indicating that the pressure is adequate. If it is a high value, the system activates a red light indicator and the acoustic indicator generates a high frequency sound, indicating that the pressure is excessive. All these limits and acoustic frequencies can be configured in the microprocessor control system (14), so that any desired reconfiguration of the system is more flexible and less costly.

The same luminous indicator (17) is capable of producing 3 colors, depending on the signal that is applied to its terminals, that in turn depends on the measured pressure.

The acoustic indicator (19) is a small but powerful loudspeaker that picks up a square-frequency signal generated by the microprocessor control system (14) when it needs to be activated.

The square control signal from the microprocessor control system (14) passes through power drivers (16,18) which amplify the signal of the microprocessor so that it is powerful enough to activate the luminous and the acoustic indicators (17,19).

The control signal that activates the accelerator (21) is generated by the microprocessor control system (14), only in the case that the pressure is adequate (green indicator and medium frequency sound). In all other cases, the signal is not activated and the accelerator (21) will be on standby.

Therefore, as represented in Figure 5, the electron accelerator (21) is directly controlled by the contact sensor (12) through the microprocessor control system (14), such that an enhanced radiotherapy treatment with maximum reliability is achieved due to the synchronization with the electron accelerator, based on direct transmission of the signal received from the contact sensor (12) to the electron accelerator (21).

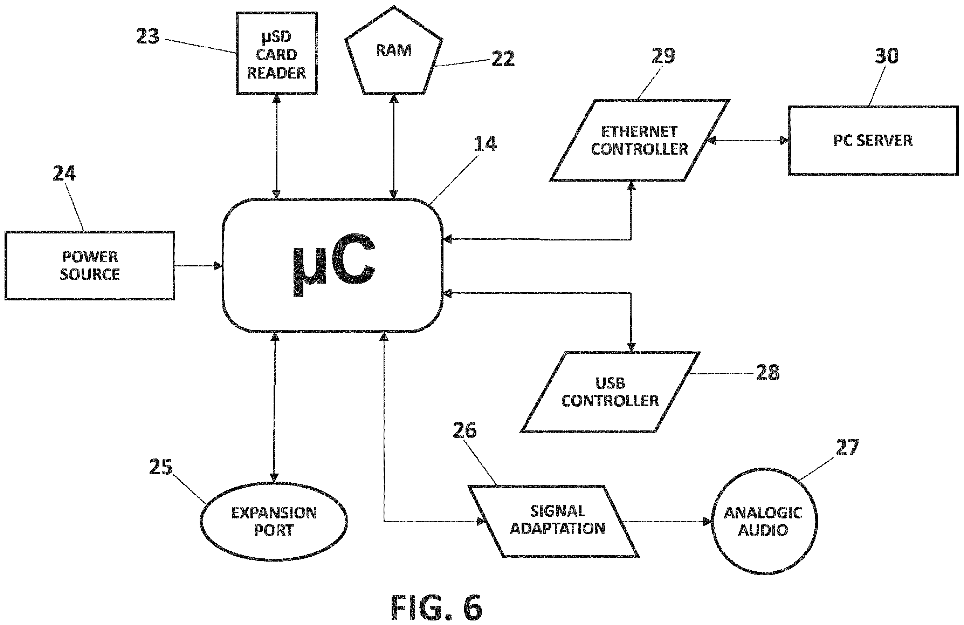

As shown in **Figure 6****,** the microprocessor control system or processor (14) is powered by a power source (24), and it is communicated with a RAM memory (22), and with a digital expansion port (25) and with a card reader (23). Furthermore, the processor (14) is communicated with a USB controller (28), and with an Ethernet controller (29) that in turn is communicated with a PC server (30).

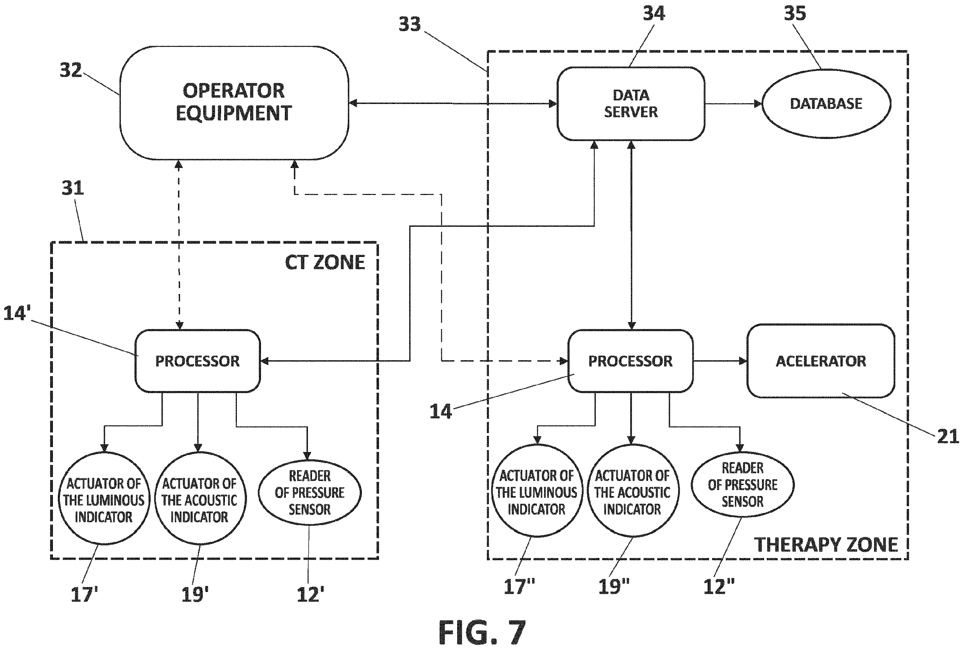

**Figure 7** shows a general electric diagram of the therapy zone (33) (accelerator) and the CT (simulation zone (31)), controlled by an operator equipment (32).

The therapy zone (33) incorporates the electronic system of **Figure 5** including the processor (14) communicated with a contact sensor reader (12") and with the radiation emission equipment (21), as well as with actuators (17",19") of the luminous and acoustic indicators (17,19) respectively. The therapy zone (33) further includes a data server (34) associated with a data base (35) that contains the patient's treatment data.

In turn, the simulation CT zone (31) includes a processor (14'), another actuator (17') of the luminous indicator (17), and an another actuator (19') of the acoustic indicator (19). The processor (14') is also communicated with a contact sensor reader (12'), and with the operator equipment (32).

In a preferred embodiment of the invention, only one electronic circuit is used to implement the electric diagram of **Figure 7****,** such that the electronic circuit can be moved to the therapy zone (33) or to the simulation CT zone (31).

The data server (34) is communicated both with the processors (14,14'), and with the operator equipment (32).

With this architecture, an operator can control and supervise through the operator equipment (32), the operation of the therapy zone (33) and the simulation CT zone (31), based on the pressure measurement provided by the pressure sensor.

The electrical system of the invention is versatile in the sense that, if the patient's system that synchronizes with the electron accelerator fails, the one used in the simulation CT zone can also be used for the synchronization, and vice versa.

Other preferred embodiments of the present invention are described in the appended dependent claims and the multiple combinations of those claims.

## Claims

1. System (1) for positioning patients for a radiation emission equipment, comprising:
a table support (3) configured to be attached to a table, and an arm (2) coupled with the table support (3),
a bar (4) mounted with the arm (2) and displaceable with respect to the arm (2), wherein the bar (4) has a contact sensor (12) adapted to be activated by the thoracic expansion of a patient lying on a table when the arm (2) is attached to a table by means of the table support (3),
signalling means for a patient lying on the table,
a control unit communicated with the contact sensor (12) and with the signalling means (17,19),
wherein the control unit is adapted to operate the signalling means as to inform a patient lying on the table as to whether the contact sensor (12) is properly activated or not, such as a patient can maintain a proper deep inspiration breath hold position,
and wherein the system (1) is adapted to be communicated with a radiation emission equipment, and to control the equipment depending on whether the contact sensor (12) is properly activated or not, such that when the system (1) is operatively communicated with a radiation emission equipment, the operation of the radiation emission equipment is controlled by the contact sensor (12), so that radiation can be applied on a patient only when the contact sensor (12) is properly activated.

2. System according to claim 1, wherein the contact sensor (12) is a pressure sensor, and wherein a pressure range is stored in the control unit, such that the contact sensor (12) is properly activated when the pressure exerted on the contact sensor (12) is within the predefined pressure range within which it is considered that the patient is in proper deep inspiration breath hold position.

3. System according to claim 1, wherein the bar (4) is generally parallel to a table when the arm (2) is coupled to the table by means of the table support (3).

4. System according to claim 1 wherein the signalling means comprises an acoustic indicator (19) and/or a luminous indicator (17).

5. System according to any of the preceding claims, wherein the bar (4) has a trapezoidal cross-sectional configuration.

6. System according to claim 5, wherein the contact sensor (12) is provided on the lower base of the trapezoidal bar (4).

7. System according to any of the preceding claims, further comprising electron accelerator or therapy zone (33) and a simulation CT zone (31) controlled by an operator equipment (32), such that an operator can control and supervise through the operator equipment (32), the operation of the simulation CT zone (31), based on a reading of the contact sensor (12).

8. System according to claim 7, wherein the control unit comprises a processor (14), in the therapy zone (33) communicated with a contact sensor reader (12"), and with an actuator (17") of the luminous indicator (17), and with an actuator (19") of the acoustic indicator (19), and wherein the therapy zone (33) further includes a data server (34) associated with a data base (35) that contains patient's treatment data.

9. System according to claim 7 or 8, wherein the simulation CT zone (31) includes a second processor (14'), another actuator (17') of the luminous indicator (17) and an another actuator (19') of the acoustic indicator (19), wherein the second processor (14') is also communicated with a contact sensor reader (12'), and with the operator equipment (32).

10. System according to claim 8, wherein the data server (34) is communicated both with the first and second processors (14,14'), and with the operator equipment (32).

11. System according to claims 2 and 8, wherein the control unit is adapted to operate the actuators (17', 17", 19', 19") of the luminous and acoustic indicators (17,19), depending on pressure readings obtained from the contact sensor (12).
